# EUROPEAN PATENT SPECIFICATION

(11) **EP 2 485 756 B1**
(45) Date of publication and mention of the grant of the patent: **20.05.2015**
(21) Application number: 10762684.8
(22) Date of filing: 07.10.2010
(51) Int. Cl.: A61K 38/26, A23L 1/00, A61P 21/00, A61K 38/27, A61K 38/17, A61K 38/22

(54) **GLP-2 for use in the treatment of muscle atrophy**
GLP-2 zur Verwendung zur Behandlung von Muskelatrophie
GLP-2 pour son utilisation dans le traitement de l'atrophie musculaire

(30) Priority: 07.10.2009 EP 09172398
(43) Date of publication of application: 15.08.2012
(73) Proprietor: Nestec S.A., 1800 Vevey (CH); I.N.R.A., 75338 Paris (FR)
(72) Inventor: ATTAIX, Didier, F-63210 Orcival (FR); CODRAN-RAISON, Audrey, F-63270 Vic Le Comte (FR); CORTHESY-THEULAZ, Irène, CH-1066 Epalinges (CH); BREUILLE, Denis, CH-1010 Lausanne (CH); COMBARET, Lydie, F-63100 Clermont-Ferrand (FR)
(74) Representative: Stephen, Paula-Marie
(86) International application number: PCT/EP2010/065021
(87) International publication number: WO 2011/042501

(56) References cited:
- WO-A1-2008/056155
- US-A- 5 661 123
- US-A1- 2003 207 809
- US-A1- 2004 052 862
- US-A1- 2004 248 782
- US-A1- 2006 105 954
- SHIN ET AL: "Mucosal Adaptation to Enteral Nutrients is Dependent on the Physiologic Actions of Glucagon-Like Peptide-2 in Mice", GASTROENTEROLOGY, ELSEVIER, PHILADELPHIA, PA, vol. 128, no. 5, 1 May 2005 (2005-05-01), pages 1340-1353, XP005314557, ISSN: 0016-5085
- JEPPESEN ET AL: "Glucagon-like peptide 2 improves nutrient absorption and nutritional status in short-bowel patients with no colon", GASTROENTEROLOGY, ELSEVIER, PHILADELPHIA, PA, vol. 120, no. 4, 1 March 2001 (2001-03-01) , pages 806-815, XP005861557, ISSN: 0016-5085
- CUOCO L ET AL: "Skeletal muscle wastage in Crohn's disease: A pathway shared with heart failure?", INTERNATIONAL JOURNAL OF CARDIOLOGY, ELSEVIER SCIENCE PUBLISHERS, AMSTERDAM, NL, vol. 127, no. 2, 4 July 2008 (2008-07-04), pages 219-227, XP022796017, ISSN: 0167-5273 [retrieved on 2008-06-28]

### SEQUENCE LISTING

<110> Nestec S.A.
<120> The intestine and muscle recovery
<130> NO 10200
<160> 4
<170> PatentIn version 3.1
<210> 1
   <211> 33
   <212> PRT
   <213> Homo sapiens
<400> 1
   His Ala Asp Gly Ser Phe Ser Asp Glu Met Asn Thr Ile Leu Asp Asn
   1               5                   10                  15
   Leu Ala Ala Arg Asp Phe Ile Asn Trp Leu Ile Gln Thr Lys Ile Thr
               20                  25                  30
   Asp
<210> 2
   <211> 33
   <212> PRT
   <213> Artificial
<220>
   <223> Human GLP-2 sequence with an Ala-->Gly exchange in position
   2
<400> 2
   His Gly Asp Gly Ser Phe Ser Asp Glu Met Asn Thr Ile Leu Asp Asn
   1               5                   10                  15
   Leu Ala Ala Arg Asp Phe Ile Asn Trp Leu Ile Gln Thr Lys Ile Thr
               20                  25                  30
   Asp
<210> 3
   <211> 33
   <212> PRT
   <213> Rattus rattus
<400> 3
   His Ala Asp Gly Ser Phe Ser Asp Glu Met Asn Thr Ile Leu Asp Asn
   1               5                   10                  15
   Leu Ala Thr Arg Asp Phe Ile Asn Trp Leu Ile Gln Thr Lys Ile Thr
               20                  25                  30
   Asp
<210> 4
   <211> 33
   <212> PRT
   <213> Artificial
<220>
   <223> Rat GLP-2 sequence with an Ala-->Gly exchange in position 2
<400> 4
   His Gly Asp Gly Ser Phe Ser Asp Glu Met Asn Thr Ile Leu Asp Asn
   1               5                   10                  15
   Leu Ala Thr Arg Asp Phe Ile Asn Trp Leu Ile Gln Thr Lys Ile Thr
               20                  25                  30
   Asp

## Description

The present invention generally relates to the field of nutrition and health. In particular, the present invention provides a composition that allows it to treat, limit or prevent skeletal muscle atrophy. Embodiments of the present invention are directed at GLP-2 containing compositions for use in the treatment or prevention of skeletal muscle atrophy.

Muscle wasting in catabolic states is an indirect cause of morbidity and mortality because the ability of the organism to recover from such catabolic events is strongly impaired.

Therefore, a major challenge in clinical nutrition is to improve whole-body protein balance, and hence protein balance in skeletal muscle, which is the major reservoir of body proteins. However, anabolic molecules such as insulin, branched-chain amino acids, or glutamine have minor if any positive effects on muscles in cachectic patients.

It was recently shown in young starved/refed rats that skeletal muscle ubiquitin-proteasome-dependent proteolysis is poorly responsive to increases in both plasma insulin and amino acid levels. This conclusion is based on *in vitro* measurements of muscle proteolysis ± proteolytic inhibitors (including the proteasome inhibitor MG132), on gene expression of several components of the ubiquitin-proteasome pathway, and on rates of ubiquitination in skeletal muscle (Kee, A.J., et al., 2003, Journal of Physiology 546:765-776).

If a decrease in the mass of a muscle occurs, then this condition is commonly named "muscle atrophy". Muscle atrophy is characterized by a partial loss of muscle mass. This muscle atrophy is related either to decrease protein synthesis or to increase protein degradation or to both of them. The consequence of muscle atrophy is usually an impaired quality of life. Daily tasks are becoming more and more difficult to perform and sudden weaknesses may cause accidents. Subjects suffering from muscle atrophy are often elderly subjects and/or subjects suffering from disorders that have shrinking muscle mass as a consequence.

Functional electrical stimulation or simply exercise is typical measures to work against muscle atrophy. However, depending on the general condition of the subject to be treated, these methods may not always be applicable.

It would hence be desirable to have available a composition which allows it to treat or prevent muscle atrophy and which also can be used in subjects that are unable to increase their physical activity.

Consequently, it was the object of the present invention to improve the state of the art and to provide an ingestible composition for treating or preventing skeletal muscle atrophy.

The present inventors achieved this object by providing the subject matter of the independent claims. The dependent claims further develop the present invention.

The inventors have performed an *in vivo* experiment to study the effect of GLP-2 administration on protein metabolism in young rats in a starvation/refeeding model.

It was found that starvation-induced small intestine atrophy in young rats is associated with increased cathepsin and proteasome activities. A GLP-2-induced increase in small intestine mass was associated with reduced cathepsin activities both in the jejunum and the ileum, whereas peptidase activities of the proteasome were not modified. The GLP-2 treatment of starved rats (i) reduced small intestine atrophy, (ii) prevented the increased activity of some cathepsins, and (iii) limited the decrease in villus height and crypt depth of the jejunum.

Finally, GLP-2 administration did accelerate the recovery of both small intestine and skeletal muscle masses when fasted rats were refed for 6h or 24h.

In this present work the atrophy of the small intestine induced by starvation was reduced by 16% when rats received GLP-2. The small intestine totally recovered after 24h of refeeding in GLP-2 treated rats, whereas 26% of atrophy still prevailed in untreated animals.

Importantly, the skeletal muscle mass recovery paralleled the small intestinal recovery, and was accelerated in GLP-2-treated rats. Skeletal muscles are likely not to carry GLP-2 receptors. Thus, altogether these observations show that the manipulation of the small intestinal mass has a beneficial impact on skeletal muscle mass.

In addition, manipulating the mass of viscera has an impact on muscle mass. The inventors presently believe that both, intestinal and liver protein masses, are highly labile and rapidly degraded by the lysosomal pathway, so that manipulating liver mass as manipulating small intestinal mass will have an impact on muscle mass.

To the inventors best knowledge was this the first study demonstrating that manipulating the small intestine mass has a beneficial impact on skeletal muscle masses, for example during recovery periods following catabolic conditions such as fasting.

These findings may be applied in all conditions where it is desired to accelerate small intestine and/or skeletal muscle recovery, for example in clinical situations.

The present work also shows that there is a prioritization in the anabolic effect of nutrition: the recovery of the intestine always precedes the recovery of muscle mass.

Consequently, the present invention relates to a composition that supports the recovery of the intestine for treating or preventing skeletal muscle atrophy.

It also relates to the use of a composition that supports the recovery of the intestine for the preparation of a composition to treat or prevent skeletal muscle atrophy.

Compositions that support the recovery of the intestine are known to the skilled artesian.

For example, a composition that supports the recovery of the intestinal mass may be a composition comprising intestinal trophic factors. Intestinal trophic factors are known to those skilled in the art and comprise growth hormones (GH), and/or vasoactive intestinal peptide (VIP), for example.

Further, a composition that supports the recovery of the intestinal mass may be a composition comprising glucagon-like peptide 2 (GLP-2) and/or a composition that stimulates the secretion of GLP-2 in a body. Consequently, the present invention relates to a composition comprising glucagon-like peptide 2 (GLP-2) for treating, or preventing muscle atrophy.

The present invention also relates to the use of glucagon-like peptide 2 (GLP-2) for the preparation of a composition to treat or prevent muscle atrophy.

Glucagon-like peptide 2 (GLP-2) is a 33-amino acid peptide derived from the tissue-specific, post-translational processing of the proglucagon gene expressed in the intestinal enteroendocrine L-cells.

Drucker et al, Gut, 2002, 50, 428-435 and Burrin et al. Journal of Nutrition, 2001, pages 709-712, report that there is relatively high homology (87-97%) in the GLP-2 peptide sequence among the species, including humans, pigs, cows and rats. Hence, the GLP-2 of the present invention includes human GLP-2 and proteins with at least 87% sequence homology to human GLP-2, preferably at least 90 % sequence homology to human GLP-2, for example at least 95 % sequence homology to human GLP-2.

Human GLP-2 has the following sequence: HADGSFSDEMNTILDNLAARDFINWLIQTKITD (SEQ-ID No. 1)

For example the A at position 2 may be replaced by a G to prevent degradation of GLP-2 by dipeptidyl peptidase IV (DP IV) and thus to increase its half-life. The resulting GLP-2 has the following sequence: HGDGSFSDEMNTILDNLAARDFINWLIQTKITD (SEQ-ID No. 2)

Further GLP-2 proteins that may be used for example in accordance with the present invention are the following: HADGSFSDEMNTILDNLATRDFINWLIQTKITD (SEQ-ID No. 3), or HGDGSFSDEMNTILDNLATRDFINWLIQTKITD (SEQ-ID No. 4).

An embodiment of the present invention relates also to a composition comprising glucagon-like peptide 2 (GLP-2) for use in the treatment, limitation and/or prevention of skeletal muscle atrophy.

There is also described a composition that stimulates the secretion of GLP-2 in a body for treating or preventing muscle atrophy.

The primary stimulus for GLP-2 secretion is nutrient intake. Xiao, Q., et al. report in Gastroenterology 117: 99-105 (1999) that N-IR-GLP-2 levels were found to increase after mixed meals and in - in particular - in response to carbohydrate and fat.

A composition that stimulates the secretion of GLP-2 in a body may hence be a composition rich in carbohydrates and/or fat.

Such a composition may have a caloric density in the range of 0.8-2.0kcal/ml with at least 10% of the calories resulting from fat and/or at least 25% of the calories resulting from carbohydrates. The composition may or may not comprise a protein source. The protein source - if present - will provide at most 10% of the calories of the composition.

The composition of the present invention may contain at least one protein source, at least one carbohydrate source and/or at least one lipid source.

If the composition contains a carbohydrate source, the kind of carbohydrate to be used is not particularly limited. Any suitable carbohydrate may be used, for example sucrose, lactose, glucose, fructose, corn syrup solids, maltodextrins, starch and mixtures thereof. Combinations of different carbohydrate sources may be used. The carbohydrate source may be present in the composition in an amount of 7.0-19.0 g/100kcal of the composition.

If the composition contains a lipid source, the kind of lipid to be used is not particularly limited. Long chain n-3 and/or n-6 polyunsaturated fatty acids, such as DHA, ARA and/or EPA may be added. A suitable fat profile may be obtained using a blend of canola oil, corn oil, high-oleic acid sunflower oil and medium chain triglyceride oil. The lipid source may be present in the composition in an amount of 1.5-5.0 g/100kcal of the composition.

If present, the protein source may contain any dietary protein, for example animal proteins (such as milk proteins, meat proteins and egg proteins); vegetable proteins (such as soy proteins, wheat proteins, rice proteins, and pea proteins); peptides; mixtures of free amino acids; or combinations thereof.

Milk proteins such as casein and whey, and soy proteins may be preferred. As far as whey proteins are concerned, the protein source may be based on acid whey or sweet whey or mixtures thereof and may include alpha- lactalbumin and betalactoglobulin in whatever proportions are desired. The protein source may also include bovine serum albumin, acid casein, caseinates, α-casein, β-casein, γ-casein. The protein source may be present in the composition in an amount of 2.0 -8.5 g/100kcal of the composition.

The proteins may be intact or hydrolysed or free amino acids. A mixture of intact and/or hydrolysed proteins and/or free amino acids may be used. It may be desirable to supply partially hydrolysed proteins (degree of hydrolysis (DH) between 2 and 20%) to facilitate absorption.

If hydrolysed proteins are required, the hydrolysis process may be carried out as desired and as is known in the art. For example, a whey protein hydrolysate may be prepared by enzymatically hydrolysing the whey fraction in one or more steps.

Dietary fibre may be added as well. They may be soluble or insoluble and in general a blend of the two types is preferred. Suitable sources of dietary fibre include soy, pea, oat, pectin, guar gum, arabic gum, fructooligosaccharides, galacto-oligosaccharides, sialyl-lactose and oligosaccharides derived from animal milks. A preferred fibre blend is a mixture of inulin with shorter chain fructo-oligosaccharides.

A composition for use according to the present invention may be prepared by any manner known in the art. For example, if the composition is a nutritional formula, such as clinical nutrition formula or an infant feeding formula it may be prepared by blending together a protein source, a carbohydrate source, and a fat source in appropriate proportions. If used, emulsifiers may be included in the blend. Vitamins and minerals may be added at this point but are usually added later to avoid thermal degradation. Any lipophilic vitamins, emulsifiers and the like may be dissolved into the fat source prior to blending. Water, preferably water which has been subjected to reverse osmosis, may then be mixed in to form a liquid mixture.

The liquid mixture may then be thermally treated to reduce bacterial loads. For example, the liquid mixture may be rapidly heated to a temperature in the range of about 120°C to about 140°C for about 5 seconds to about 30 seconds. This may be carried out by steam injection or by heat exchanger; for example a plate heat exchanger.

The liquid mixture may then be cooled to about 60°C to about 85°C; for example by flash cooling. The liquid mixture may then be homogenised; for example in two stages at about 7 MPa to about 40 MPa in the first stage and about 2 MPa to about 14 MPa in the second stage. The homogenised mixture may then be further cooled to add any heat sensitive components; such as vitamins and minerals.

The pH and solids content of the homogenised mixture is conveniently standardised at this point. The homogenised mixture is transferred to a suitable drying apparatus such as a spray drier or freeze drier and converted to powder. The powder should have a moisture content of less than about 5% by weight.

GLP-2 may also be co-administered with a composition that stimulates the secretion of GLP-2 in a body.

The composition for use according to the present invention may be administered to humans or animals, in particular pet animals. It may further be to be administered to patients with an intact colon.

The composition for use according to the present invention may be any kind of composition. The composition may be to be administered orally, enterally, parenterally (intravenously or subcutaneously or intramuscularly), for example.

For example it may be a pharmaceutical composition, a nutraceutical, a food additive, a pet food, a food product, or a drink.

Food products for use according to the present invention include dairy products, such as fermented milk products, e.g., yoghurts, buttermilk, etc; ice creams; concentrated milk; milk; dairy creams; flavoured milk drinks; whey based drinks; toppings; coffee creamers; chocolate; cheese based products; soups; sauces; purees; dressings; puddings; custards; baby foods; nutritional formulas, such as those for complete nutrition, for example for infants, children, teenagers, adults, the elderly or the critically ill; cereals and cereal bars, for example.

Drinks include for example milk- or yoghurt based drinks, fermented milk, protein drinks, coffee, tea, energy drinks, soy drinks, fruit and/or vegetable drinks, fruit and/or vegetable juices.

A food additive or a medicament may be in the form of tablets; capsules; pastilles; sachets; gels; or liquids, e.g., nutritional solutions; for example.

The compositions may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellifying agents, gel forming agents, antioxidants and antimicrobials. They may also contain conventional pharmaceutical additives and adjuvants, excipients and diluents, including, but not limited to, water, gelatine of any origin, vegetable gums, ligninsulfonate, talc, sugars, starch, gum arabic, vegetable oils, polyalkylene glycols, flavouring agents, preservatives, stabilizers, emulsifying agents, buffers, lubricants, colorants, wetting agents, fillers, and the like.

Further, the compositions may contain an organic or inorganic carrier material suitable for oral or enteral administration as well as vitamins, minerals trace elements and other micronutrients in accordance with the recommendations of Government bodies such as the USDA.

A stabilizing agent may be added to stabilize the composition and its constituents.

A flavouring agent and/or a colouring agent may be added to adjust flavours and to give the composition a colour that is easy to identify and/or that is perceived as pleasant.

The compositions for use according to the present invention may be used to improve skeletal muscle recovery. This may be needed in particular in the field of clinical nutrition.

The compositions of the present invention may also be used to improve small intestine and skeletal muscle mass recovery.

Importantly, the recovery of the small intestine and of skeletal muscle mass may be improved simultaneously by using the compositions described in the present invention.

The compositions for use according to the present invention may also be for use in increasing liver protein mass regain.

Consequently, this combined effect may be achieved by administering a composition comprising glucagon-like peptide 2 (GLP-2).

The compositions for use according to the present invention may be to be administered during or after a period of malnutrition.

A period of malnutrition may - for example - be caused by several clinical conditions such as various acute or chronic disorders, e.g., infection, cancer, AIDS, burn injury, trauma, and other. Malnutrition may of course also follow from insufficient food intake or from the consumption of a diet that does not supply all nutrients in sufficient amounts.

Those of skill in the art will understand that all features described with respect to present invention may be combined.

Further features and advantages of the present invention are apparent from the following examples, tables and figures.

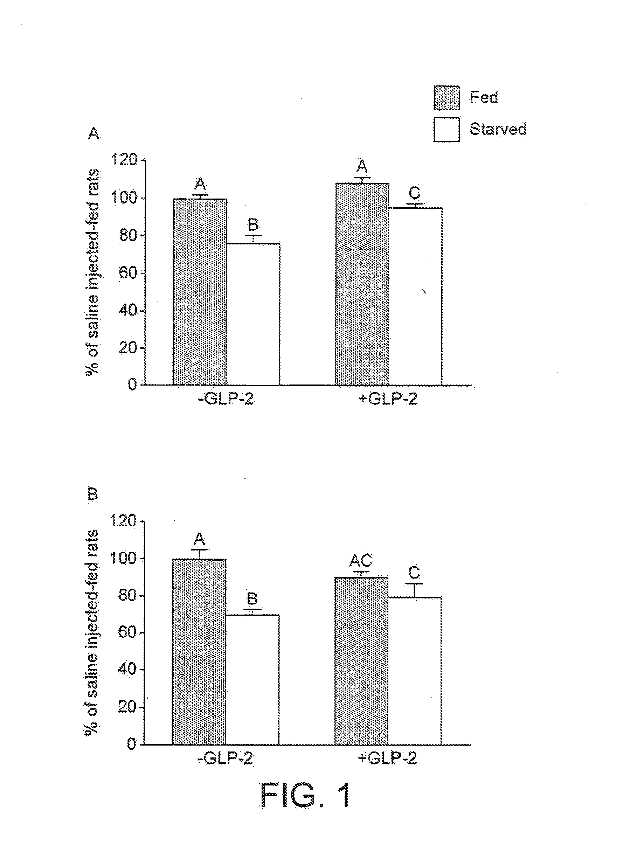

Figure 1. Morphometric analysis and measurements of villus height (A) and crypt depth (B) in the jejunum. Values are means ± SEM (vertical bars) for n=5 animals and are expressed as of fed controls. Fed rats (closed bars); starved rats (open bars). Bars with different letters are significantly different (P < 0.05).

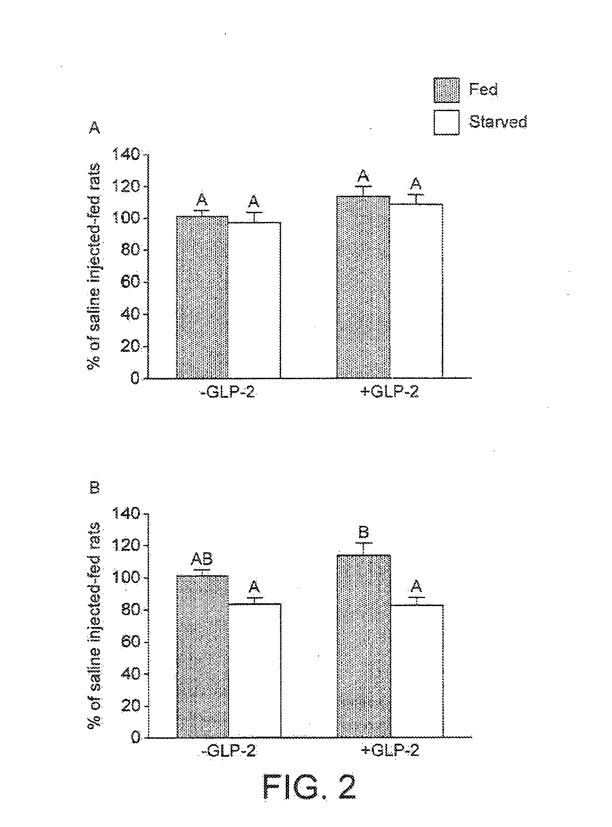

Figure 2. Morphometric analysis and measurements of villus height (A) and crypt depth (B) in the ileum. Values are means ± SEM (vertical bars) for n=5 animals and are expressed as % of fed controls. Fed rats (closed bars), starved rats (open bars). Bars with different letters are significantly different (P < 0.05).

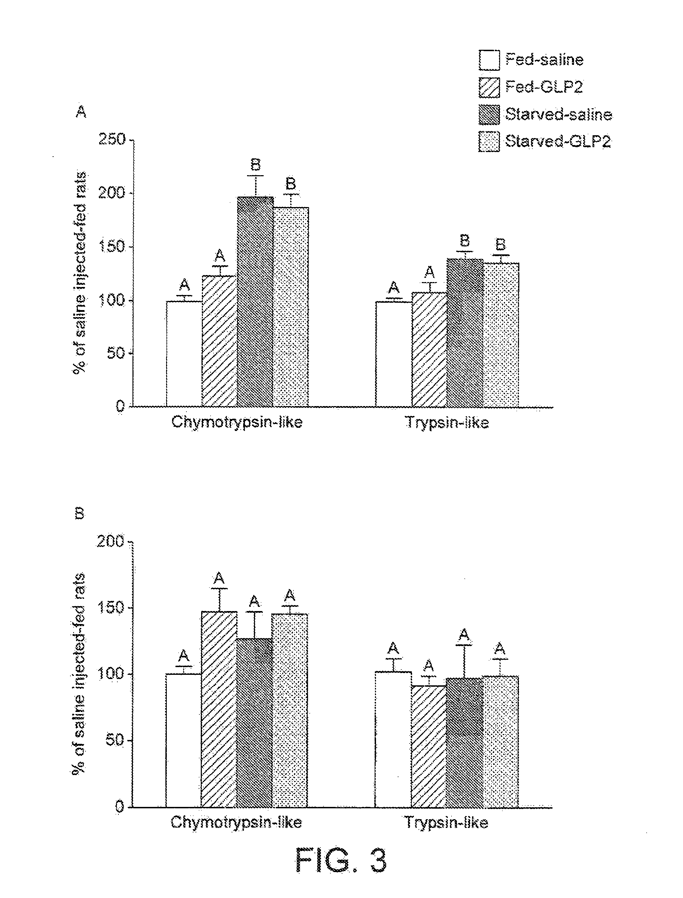

Figure 3. Chymotrypsin- and trypsin-like activities of the proteasome in the jejunum (A) and the ileum (B) from saline- or GLP-2- injected fed and starved rats. Data represent the slopes of best fit of arbitrary fluorescence units released from the fluorescent substrates (see Examples section) vs. time. Values are expressed in % of saline-injected fed rats and are means ± SEM (vertical bars) for 5 animals per group. Saline injected-fed rats (open bars), GLP-2 injected-fed rats (hatched bars), saline injected-starved rats (closed bars) and GLP-2 injected-starved rats (dot bars). Bars with different letters are significantly different (P < 0.05).

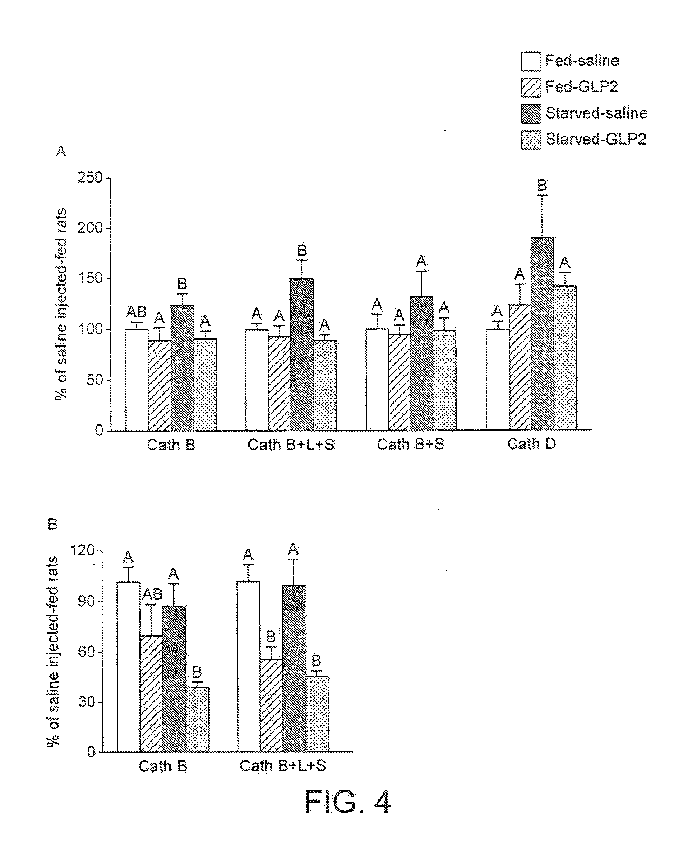

Figure 4. Cathepsin (B, B+S, B+L+S and D) activities in the jejunum (A) and the ileum (B) from saline- or GLP-2- injected fed and starved rats. Data represent the slopes of best fit of arbitrary fluorescence units released from the fluorescent substrates (see Material and methods section) vs. time. Values are expressed in % of saline-injected fed rats and are means ± SEM (vertical bars) for 5 animals per group. Saline injected-fed rats (open bars), GLP-2 injected-fed rats (hatched bars), saline injected-starved rats (closed bars) and GLP-2 injected-starved rats (dot bars). Bars with different letters are significantly different (P < 0.05).

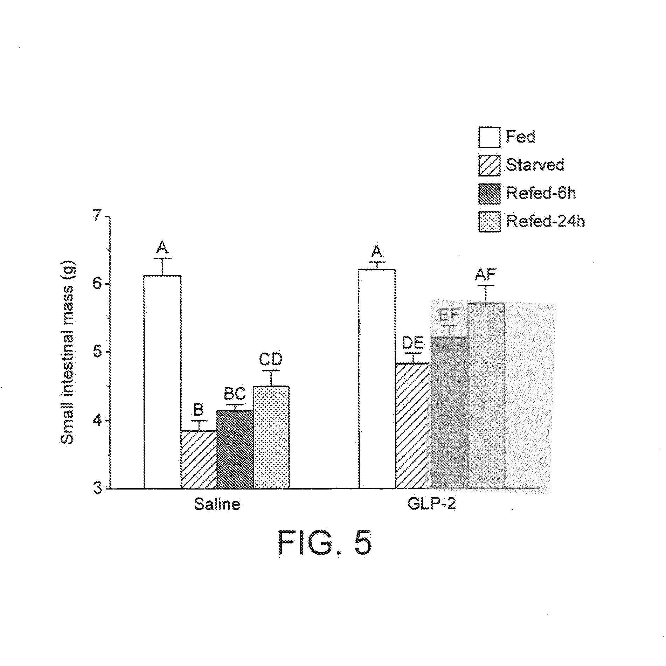

Figure 5. Small intestinal mass in saline- or GLP-2-injected rats following 48h of starvation and 6 or 24h of refeeding. Data are means ± SEM for 5 animals per group. Fed rats (open bars), starved rats (closed bars), 6h refed-starved rats (hatched bars) and 24h refed-starved rats (grey bars). Bars with different letters are significantly different (P < 0.05).

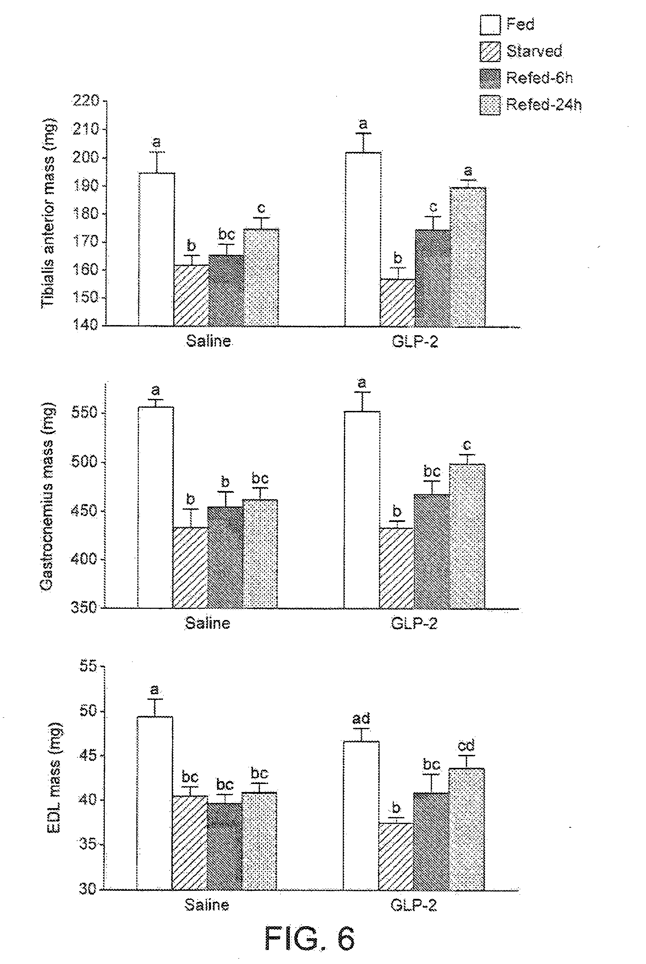

Figure 6. Tibialis anterior, gastrocnemius and EDL muscle masses in saline- or GLP-2-injected rats following 48h of starvation and 6 or 24h of refeeding. Data are means ± SEM for 5 animals per group. Fed rats (open bars), starved rats (closed bars), 6h refed-starved rats (hatched bars) and 24h refed-starved rats (grey bars). Bars with different letters are significantly different (P < 0.05).

Table 1: Body weight, small intestinal and skeletal muscle mass of fasted rats. Data are means ± SEM, n=8 rats per group. Values within a row with different superscripts are significantly different (P < 0.05).

Table 2: Enzyme activities in rat small intestine after starvation. Data are expressed in % of Fed controls and are means ± SEM, n=8 rats per group. Values within a row with different superscripts are significantly different (P < 0.05).

Table 3: Role of GLP-2 on starvation-induced body weight loss and intestine atrophy. Data are means ± SEM, n=13-15 rats per group. Statistical differences were assessed by two-way ANOVA, followed by the "a posteriori" test of PLSD Fisher's. N, starvation effect; G, GLP-2 effect. Values within a row with different superscripts are significantly different (P < 0.05).

Table 4: Role of GLP-2 on starvation-induced skeletal muscle atrophy. Data are means ± SEM, n=5 rats per group. Statistical differences were assessed by two-way ANOVA, followed by the "a posteriori" test of PLSD Fisher's. N, starvation effect; G, GLP-2 effect. Values within a row with different superscripts are significantly different (P < 0.05).

### Examples:

### Materials

Rat recombinant GLP-2 was purchased from Washington Biotechnology Inc. (Columbia, MD, USA). [Ala] in position 2 was substituted by [Gly] to make the peptide resistant to DPP IV degradation. The GLP-2 peptide sequence used in this study is as follows: HGDGSFSDEMNTILDNLATRDFINWLIQTKITD.

### Animals, Housing and Experimental Design

Young male Wistar rats (50-60 g) were obtained from Charles River (L'Arbresle, France) and maintained in a temperature controlled room (22 ± 1°C) with a 12 h light/12 h dark cycle. They were given free access to a standard rodent diet (A03; UAR, Epernay sur Orge, France) and to water during a 5 days acclimatization period. Three experiments were conducted. (1) In the first experiment, rats were fed ad libitum or fasted for 1 or 2 days. At the end of the fasting period, rats were anesthetized with intraperitoneal injection of urethane (1.7 g/kg body weight, Sigma Aldrich, Lyon, France) and sacrificed. (2) In the second experiment, animals were randomly divided into control and GLP-2 treated groups (n=26-30 per group). Injections of saline (400 µL) or GLP-2 (20 µg/400 µL of saline) every 12 h started at day 0 and continued until the end of the experiment. At day 4, half of each group was deprived of food for 2 days. At day 6, animals were sacrificed as described. (3) In the last experiment, rats were allocated as described in experiment 2. Control and GLP-2 treated groups (n=20 per group) were divided into 2 groups of fed (n=5) and starved rats (n=15). Saline and GLP-2 injections continued until the sacrifice of the rats. At day 6, 5 rats from the fed and the 48h-fasted groups treated or not with GLP-2 were sacrificed as described. The remaining fasted animals from the control and GLP-2 treated groups were refed *ad libitum* from day 6 for 6 or 24h. After refeeding, rats were sacrificed as described.

### Tissue Collection

The entire small intestine was rapidly excised, rinsed three times with ice-cold PBS, blotted and weighed. Ten cm caudal to the pyloric sphincter and ten cm cranial to the ileo-caecal junction were taken and discarded. Jejunum and ileum were excised, weighed, and divided in small pieces of 5 cm. The first piece was immediately processed for cathepsin activities. The remaining pieces were frozen in liquid nitrogen, and stored at -80°C until further analyses. In experiment 3, skeletal muscles (EDL, gastrocnemius and tibialis anterior) were also carefully dissected and weight.

### Cathepsin Activities

The five cm pieces of jejunum and/or ileum were immediately homogenized using a polytron homogenizer in a 10 mmol/L phosphate buffer (pH 7.4) containing 50 mmol/L KCl, 1 mmol/L EDTA and 0.25 mol/L sucrose as described previously.³⁰ Homogenates were then centrifuged at 1,000g (10 minutes, 4°C). The supernatant was centrifuged at 2,500g (10 minutes, 4°C) and then again at 40,000g (20 minutes, 4°C). The pellet was resuspended in 20 mmol/L sodium acetate buffer (pH 5.5) containing 0.2 mmol/L EDTA and stored at -20°C until further analysis. Cathepsin B and cathepsin B+L+S activities were determined using the fluorogenic substrates Z-arginine-arginine-AMC (ZAA-AMC, 50 µmol/L, Sigma), Z-phenylalanine-arginine-AMC (ZPA-AMC, 30 µmol/L, Sigma), respectively.³⁰ Neutral pH reducing the cathepsin L activity, the bulk of the cathepsin B+L+S activity measured with ZPA-AMC at pH 7.0 reflects the cathepsin B+S activity. The cathepsin D activity was determined using the fluorogenic substrate MOCAc-Gly-Lys-Pro-Ile-Leu-Phe-Arg-Leu-Lys (Dnp)-D-Arg-NH2 (MOCA, 20 µmol/L, Peptide Institute Inc, Osaka, Japan). The peptidase activities were determined by measuring the accumulation of the fluorogenic cleavage product (methylcoumaryl-amide, AMC) at 37°C during 45 minutes using a luminescence spectrometer FLX800 (Biotek). Fluorescence was measured continuously at 380 nm excitation and 440 nm emission wavelengths. The time course for the accumulation of AMC after hydrolysis of the substrate was analyzed by linear regression to calculate peptidase activities, e.g. the slopes of best fit of accumulated AMC. The final data were corrected by the amount of protein in the reaction.

### Proteasome Activities

Proteins from pieces of jejunum and/or ileum were homogenized using a polytron homogenizer, in ice-cold buffer (pH 7.5) containing 50 mmol/L Tris/HCl, 5 mmol/L MgCl₂, 250 mmol/L sucrose, 10 mmol/L ATP, 1 mmol/L DTT, 10 µg/mL aprotinin, 10 µg/mL leupeptin, 10 µg/mL antipain, 10 µg/mL pepstatin A, and 0.2 mmol/L phenylmethylsulphonylfluoride (PMSF). Proteasomes were isolated as described.³¹ Proteasome pellets were resuspended in buffer containing 50 mmol/L Tris/HCl, 5 mmol/L MgCl₂, and 20% glycerol. The isolated proteasomes were stored at -80°C until analysis. The protein content of the proteasome preparation was determined using the Biorad protein assay following the manufacturer instructions. The chymotrypsin-, the trypsin-like activities were determined using the fluorogenic substrates succinyl-leu-leu-val-tyr-AMC (Suc-LLVY-AMC, 300 µmol/L, Sigma) and boc-leu-arg-arg-AMC (Boc-LRR-AMC, 800 µmol/L, Biomol International LP, Exeter, Devon, UK) respectively, in the presence and in the absence of the proteasome inhibitor MG132 (40 µmol/L, Sigma). The accumulation of AMC at 37°C during 45 minutes was measured continuously as described for cathepsin activities measurements. The difference between arbitrary fluorescence units recorded with or without 40 µmol/L □of the proteasome inhibitor MG132 (Affiniti) in the reaction medium was calculated. The final data were corrected by the amount of protein in the reaction.

### Histologic and Morphometric Measurements

Tissue samples from jejunum and ileum were fixed in 10% formaldehyde buffered with Na₂CO₃ (10 g/L) and processed for villus height and crypt depth measurements. Treatment designation of slides was blinded at the time of measurement.

### Statistics

Data are expressed as means ± SEM. One-way or two-way ANOVA were used to address for fasting effect in experiment 1 and for fasting and GLP-2 effects experiment 2 and 3. A posteriori PLSD Fisher's test was performed in order to assess for differences among groups. P values < 0.05 were considered statistical significance and P values < 0.10 were considered statistical tendency.

### Results

Small intestine peptidase activities following fasting Table 1 shows that fasting induced a strong and progressive loss of body weight following 1 and 2 days of food deprivation (-16% and -28%, respectively). The mass of the small intestine and the EDL muscle decreased progressively until 2 days of starvation to reach 27% of atrophy (Table 1).

To characterize the role of proteolysis in the small intestine atrophy, peptidase activities from the lysosomal and the proteasome dependent proteolytic pathways were assessed in the jejunum (Table 2).

Starvation induced an increase of the chymotrypsin- (+47%, ns), the trypsin- (+66%), and the caspase-like (+111%) activities of the 20S proteasome. In addition, cathepsin B, B+S, and B+L+S activities increased by 34 to 38% in 48h starved rats compared to fed animals. Cathepsin D activity increased as soon as 24h of food deprivation (+91%) and still rose up to 161% after 48h of fasting. The atrophy of the small intestine is thus correlated with an elevation of cathepsin and of proteasome activities in the jejunum with a maximal effect following 48h of food deprivation. The effects of GLP-2 on small intestinal proteolysis were thus studied on fed and 48h starved rats.

### Effect of GLP-2 on starvation-induced body weight loss, small intestine atrophy and histological modifications

The daily food intake of animals was not modified by the GLP-2 treatment (data not shown). Starvation induced a strong loss of body weight which was similar between untreated and treated groups (-25% and -22% respectively, P<0.0001, Table 3). The small intestine was strongly atrophied following starvation (-29, P<0.0001) and the atrophy was similar in the jejunum and in the ileum (-32 and -22% respectively, P<0.001, table 3).

Two-way ANOVA analysis indicated that, in addition to the atrophy induced by the starvation, GLP-2 treatment increased total small intestinal, jejunal and ileal mass by 22% (P<0.0001), 21% (P<0.0001) and 17.5% (P<0.005), respectively. Although, the effect of starvation was similar in saline and GLP-2 injected rats, the whole small intestine and the ileum mass was similar in saline injected-fed rats and in GLP-2 treated-starved rats. In addition, GLP-2 limited the atrophy of the jejunum in the GLP-2-treated starved rats (-12% vs. saline injected-fed rats, P<0.005) compared with the untreated starved rats (-32% vs. saline injected-fed rats, P<0.0001) (Table 3). Fasting induced a decrease of villus height and crypt depth in the jejunum (-23% and -31% respectively, P<0.05, Fig. 1). On the opposite, starvation induced only few morphological changes in the ileum (Fig. 2). In the jejunum, GLP-2 treatment limited the reduction of the villus height (-13%) and blocked the decrease of crypt depth following starvation (Fig. 1). The effects of GLP-2 treatment were quite different in the ileum since it had no effect on the villus height and did not inhibit the reduction of crypt depth following fasting (Fig. 2).

### Effect of GLP-2 on starvation-induced elevation of peptidase activities of the proteasome in the small intestine

In experiment 2, starvation induced a strong increase of chymotrypsin-like (+ 98%, P<0.0001) and trypsin-like (+40%, P<0.0005) activities of the proteasome in the jejunum (Fig. 3A) but had no effect on these activities in the ileum (Fig. 3B) in saline injected rats. Peptidase activities of the proteasome in the jejunum (Fig. 3A) or in the ileum (Fig. 3B) are not modified by the daily administration of GLP-2. Moreover, GLP-2 treatment did not block the increase of the chymotrypsin- and the trypsin-like activities seen in the jejunum following fasting (Fig. 3A).

Lysosomal proteolytic activities were measured in both the jejunum and the ileum. Cathepsin B+L+S and D activities were increased following starvation in the jejunum by 25% (P<0.01) and 90% (P<0.05), respectively (Fig. 4A). In addition, cathepsin B and B+S activities tended to increase respectively by 50% (P<0.10) and 31% (P>0.1) in the jejunum of starved rats. By contrast, cathepsin B and B+L+S did not increase at all in the ileum of starved rats compared with fed controls (Fig. 4B). Interestingly, GLP-2 treatment blocked the increase in cathepsin activities in the jejunum of starved rats (Fig 4A). Furthermore, GLP-2 administration induced a decrease of cathepsin B (-43%, P<0.002) and B+L+S (-50%, P<0.0005) activities in the ileum compared with untreated rats, even starvation did not modify the lysosomal activities in this tissue (Fig. 4B). These results indicated that the GLP-2 protective effects on the jejunum may result from an inhibition of the lysosomal pathway.

### Effect of GLP-2 on starvation-induced skeletal muscle atrophy

The tibialis anterior, the gastrocnemius and the EDL muscles atrophied by ∼20% (P<0.0001) following 2 days of starvation. GLP-2 administration did not prevent this skeletal muscle loss. However, GLP-2 is believed to be a physiologic regulator of the dynamic adaptation of the gut mucosal epithelium in response to luminal nutrients. Thus, the impact of GLP-2 treatment has been studied in refed rats following the 48h starvation period. Figure 5 showed that GLP-2 treated animals exhibited an accelerated recovery of the small intestinal mass compared to saline injected animals. The small intestinal atrophy was reduced to 15% (P<0.001) after 6h and totally absent after 24h of refeeding when rats received the GLP-2, while it still prevailed both at 6h (-32%, P<0.0001) and 24h (-26%, P<0.0001) in saline injected rats. Interestingly, skeletal muscle mass recovery paralleled the small intestine recovery and was improved by the treatment. The tibialis anterior (Fig. 6A), the gastrocnemius (Fig. 6B) and the EDL (Fig. 6C) muscles were still atrophied respectively by 10%, 16.5% and 18% (P<0.0001) after 24h of refeeding in saline injected rats. This was almost totally identical to the muscle mass loss observed after 48h of starvation (Fig. 6). By contrast, GLP-2 treatment enhanced the skeletal muscle recovery. Tibialis anterior and EDL muscles totally recovered after 24h of refeeding, whereas the gastrocnemius was still slightly atrophied by 9% (P<0.05) compared to GLP-2 treated fed rats.

**Table 1:**

| | Fed | 1-day fasted | 2-days fasted |
|---|---|---|---|
| Body weight (g) | 96.3 ± 2.2^{a} | 80.6 ± 1.9^{b} | 68.5 ± 1.0^{c} |
| Small intestinal weight (g) | 5.48 ± 0.21^{a} | 4.40 ± 0.16^{b} | 4.03 ± 0.13^{b} |
| EDL (mg) | 43.8 ± 1.3^{a} | 38.1 ± 0.9^{b} | 32.0 ± 0.6^{c} |

**Table 2:**

| | Jejunum | | |
|---|---|---|---|
| | Fed | 1-day starved | 2-days starved |
| *Proteasome* | | | |
| Chymotrypsin-like | 100 ± 24.5^{a} | 143.9 ± 20.2^{a} | 146.6 ± 16.5^{a} |
| Trypsin-like | 100 ± 159^{a} | 142.7 ± 11.2^{b} | 165.8 ± 17.5^{b} |
| Caspase-like | 100 ± 24.0^{a} | 146.4 ± 28.3^{a} | 210.6 ± 36.2^{b} |
| | | | |

| *Lysosomal* | | | |
|---|---|---|---|
| Cathepsin B | 100 ± 6.9^{a} | 124.6 ± 8.9^{ab} | 135.5 ± 10.7^{b} |
| Cathepsin B+S | 100 ± 11.0^{a} | 119.9 ± 15.7^{ab} | 134.3 ± 11.8^{b} |
| Cathepsin B+L+S | 100 ± 15.5^{a} | 115.7 ± 14.5^{ab} | 138.2 ± 12.4^{b} |
| Cathepsin D | 100 ± 10.8^{a} | 191.5 ± 20.7^{b} | 261.2 ± 24.6^{c} |

**Table 3:**

| | Fed | | Starved | | |
|---|---|---|---|---|---|
| | Saline | GLP-2 | Saline | GLP-2 | |
| Body weight (g) | 112.6 ± 2.3^{a} | 112.5 ± 1.7^{a} | 84.4 ± 1.1^{b} | 87.2 ± 1.0^{b} | N |
| Small intestine mass (g) | 4.88 ± 0.22^{a} | 5.57 ± 0.17^{b} | 3.47 ± 0.11^{c} | 4.56 ± 0.11^{a} | N, G |
| Jejunum mass (g) | 2.97 ± 0.11^{a} | 3.45 ± 0.08^{b} | 2.02 ± 0.06^{c} | 2.62 ± 0.09^{d} | N, G |
| Ileum mass (g) | 1.09 ± 0.07^{ab} | 1.24 ± 0.07^{b} | 0.85 ± 0.04^{c} | 1.02 ± 0.05^{a} | N, G |

**Table 4:**

| | Fed | | Starved | | |
|---|---|---|---|---|---|
| | Saline | GLP-2 | Saline | GLP-2 | |
| tibialis anterior (mg) | 191.5 ± 5.2^{a} | 195.7 ± 5.0^{a} | 156.9 ± 2.8^{b} | 156.6 ± 2.0^{b} | N |
| gastrocnemius (g) | 0.520 ± 0.015^{a} | 0.513 ± 0.013^{a} | 0.413 ± 0.010^{b} | 0.415 ± 0.006^{b} | N |
| EDL (mg) | 47.7 ± 1.3^{a} | 45.7 ± 0.9^{a} | 38.1 ± 0.8^{b} | 37.2 ± 0.9^{b} | N |

## Claims

1. Composition comprising glucagon-like peptide 2 (GLP-2) for use in the treatment, limitation and/or prevention of skeletal muscle atrophy.

2. Composition comprising GLP-2 for use according to claim 1 via supporting the recovery of the intestine.

3. Composition comprising GLP-2 for use according to one of the preceding claims, wherein the composition further comprises intestinal trophic factors, such as growth hormones (GH), and/or vasoactive intestinal peptide (VIP).

4. Composition comprising GLP-2 for use according to one of claims 1-3, wherein the GLP-2 has an amino acid sequence selected from the group consisting of HADGSFSDEMNTILDNLAARDFINWLIQTKITD (SEQ-ID No. 1), HGDGSFSDEMNTILDNLAARDFINWLIQTKITD(SEQ-ID No. 2), HADGSFSDEMNTILDNLATRDFINWLIQTKITD (SEQ-ID No. 3), HGDGSFSDEMNTILDNLATRDFINWLIQTKITD (SEQ-ID No. 4), or combinations thereof.

5. Composition comprising GLP-2 for use according to one of the preceding claims for improvement of skeletal muscle recovery.

6. Composition comprising GLP-2 for use according to one of the preceding claims for improvement of skeletal muscle mass during recovery periods following catabolic conditions.

7. Composition comprising GLP-2 for use according to one of the preceding claims, to be administered during or after a period of malnutrition.

8. Composition comprising GLP-2 for use according to claim 7 wherein the period of malnutrition is caused by infection, cancer, AIDS, burn injury, trauma, or from insufficient food intake, or from the consumption of a diet that does not supply all nutrients in sufficient amounts.

9. Composition comprising GLP-2 for use according to one of the preceding claims, wherein the composition is selected from the group consisting of a food product, a nutraceutical, a drink, a food additive and a medicament.

10. Composition comprising GLP-2 for use according to one of the preceding claims, wherein the composition is a nutritional formula.

11. Composition comprising GLP-2 for use according to one of the preceding claims, wherein the composition is intended for oral, enteral and/or parenteral administration.

12. Composition comprising GLP-2 for use according to one of the preceding claims wherein the composition comprises a carbohydrate source in an amount of 7-19 g/100kcal of the composition.

13. Composition comprising GLP-2 for use according to one of the preceding claims wherein the composition comprises a lipid source in an amount of 1.5-5.0 g/100kcal of the composition.

14. Composition comprising GLP-2 for use according to one of the preceding claims wherein the composition comprises a protein source in an amount of 2.0 -8.5 g/100kcal of the composition.

## Patentansprüche

1. Zusammensetzung aufweisend:
Glucagon-ähnliches Peptid-2 (GLP-2) zur Verwendung bei der Behandlung, Eingrenzung und/oder Vorbeugung von Skelettmuskelatrophie.

2. Zusammensetzung mit GLP-2 zur Verwendung nach Anspruch 1 *durch* Unterstützung der Rehabilitation des Darms.

3. Zusammensetzung mit GLP-2 zur Verwendung nach einem der vorhergehenden Ansprüche, wobei die Zusammensetzung weiterhin intestinale trophische Faktoren aufweist wie Wachstumshormone (GH) und/oder vasoaktives intestinales Peptid (VIP).

4. Zusammensetzung mit GLP-2 zur Verwendung nach einem der Ansprüche 1-3, wobei das GLP-2 eine Aminosäuresequenz besitzt, die aus der Gruppe ausgewählt ist, die folgendes aufweist: HADGSFSDEMNTILDNLAARDFINWLIQTKITD (SEQ-ID Nr. 1), HGDGSFSDEMNTILDNLAARDFINWLIQTKITD (SEQ-ID Nr. 2), HADGSFSDEMNTILDNLATRDFINWLIQTKITD (SEQ-ID Nr. 3), HGDGSFSDEMNTILDNLATRDFINWLIQTKITD (SEQ-ID Nr. 4) oder Kombinationen daraus.

5. Zusammensetzung mit GLP-2 zur Verwendung nach einem der vorhergehenden Ansprüche zur Verbesserung der Skelettmuskelrehabilitation.

6. Zusammensetzung mit GLP-2 zur Verwendung nach einem der vorhergehenden Ansprüche zur Verbesserung der Skelettmuskelmasse während Rehabilitationszeiträumen nach katabolischen Erkrankungen.

7. Zusammensetzung mit GLP-2 zur Verwendung nach einem der vorhergehenden Ansprüche, die während oder nach einem Zeitraum der Fehlernährung zu verabreichen ist.

8. Zusammensetzung mit GLP-2 zur Verwendung nach Anspruch 7, wobei der Zeitraum der Fehlernährung verursacht ist durch Infektion, Krebs, AIDS, Brandverletzung, Trauma oder durch unzureichende Nahrungsaufnahme oder durch Aufnahme einer Ernährung, die nicht alle Nährstoffe in ausreichenden Mengen liefert.

9. Zusammensetzung mit GLP-2 zur Verwendung nach einem der vorhergehenden Ansprüche, wobei die Zusammensetzung ausgewählt ist aus der Gruppe, die ein Lebensmittelprodukt, eine Nutrazeutikum, ein Getränk, einen Lebensmittelzusatz und ein Medikament aufweist.

10. Zusammensetzung mit GLP-2 zur Verwendung nach einem der vorhergehenden Ansprüche, wobei die Zusammensetzung eine Nahrungsrezeptur ist.

11. Zusammensetzung mit GLP-2 zur Verwendung nach einem der vorhergehenden Ansprüche, wobei die Zusammensetzung zur oralen, enteralen und/oder parenteralen Verabreichung bestimmt ist.

12. Zusammensetzung mit GLP-2 zur Verwendung nach einem der vorhergehenden Ansprüche, wobei die Zusammensetzung eine Kohlenhydratquelle in einer Menge von 7-19 g/100 kcal der Zusammensetzung aufweist.

13. Zusammensetzung mit GLP-2 zur Verwendung nach einem der vorhergehenden Ansprüche, wobei die Zusammensetzung eine Lipidquelle in einer Menge von 1,5-5,0 g/100 kcal der Zusammensetzung aufweist.

14. Zusammensetzung mit GLP-2 zur Verwendung nach einem der vorhergehenden Ansprüche, wobei die Zusammensetzung eine Proteinquelle in einer Menge von 2,0-8,5 g/100 kcal der Zusammensetzung aufweist.

## Revendications

1. Composition comprenant du glucagon-like peptide 2 (GLP-2) pour une utilisation dans le traitement, la limitation et/ou la prévention de l'atrophie musculaire squelettique.

2. Composition comprenant du GLP-2 pour une utilisation selon la revendication 1 par un soutien de la récupération de l'intestin.

3. Composition comprenant du GLP-2 pour une utilisation selon l'une des revendications précédentes, la composition comprenant en outre des facteurs trophiques intestinaux, comme par exemple des hormones de croissance (GH), et/ou un peptide intestinal vasoactif (VIP).

4. Composition comprenant du GLP-2 pour une utilisation selon l'une des revendications 1-3, dans laquelle le GLP-2 présente a une séquence d'acides aminés choisie parmi le groupe constitué par HADGSFSDEMNTILDNLAARDFINWLIQTKITD (SEQ-ID No. 1), HGDGSFSDEMNTILDNLAARDFINWLIQTKITD (SEQ-ID No. 2), HADGSFSDEMNTILDNLATRDFINWLIQTKITD (SEQ-ID No. 3), HGDGSFSDEMNTILDNLATRDFINWLIQTKITD (SEQ-ID No. 4), ou des combinaisons de ceux-ci.

5. Composition comprenant du GLP-2 pour une utilisation selon l'une des revendications précédentes pour une amélioration de la récupération musculaire squelettique.

6. Composition comprenant du GLP-2 pour une utilisation selon l'une des revendications précédentes pour une amélioration de la masse musculaire squelettique au cours de périodes de récupération faisant suite à des états cataboliques.

7. Composition comprenant du GLP-2 pour une utilisation selon l'une des revendications précédentes, destinée à être administrée pendant ou après une période de malnutrition.

8. Composition comprenant du GLP-2 pour une utilisation selon la revendication 7, la période de malnutrition étant causée par une infection, un cancer, le sida, une brûlure, un traumatisme, ou par un apport alimentaire insuffisant, ou par la consommation d'un régime qui ne fournit pas tous les nutriments en quantités suffisantes.

9. Composition comprenant du GLP-2 pour une utilisation selon l'une des revendications précédentes, la composition étant choisie parmi le groupe constitué par un produit alimentaire, un nutraceutique, une boisson, un additif alimentaire et un médicament.

10. Composition comprenant du GLP-2 pour une utilisation selon l'une des revendications précédentes, la composition étant une formule nutritionnelle.

11. Composition comprenant du GLP-2 pour une utilisation selon l'une des revendications précédentes, la composition étant destinée à une administration orale, entérale et/ou parentérale.

12. Composition comprenant du GLP-2 pour une utilisation selon l'une des revendications précédentes, la composition comprenant une source de glucides en une quantité de 7-19 g/100 kcal de la composition.

13. Composition comprenant du GLP-2 pour une utilisation selon l'une des revendications précédentes, la composition comprenant une source de lipides en une quantité de 1,5-5,0 g/100 kcal de la composition.

14. Composition comprenant du GLP-2 pour une utilisation selon l'une des revendications précédentes, la composition comprenant une source de protéines en une quantité de 2,0-8,5 g/100 kcal de la composition.
